# EUROPEAN PATENT SPECIFICATION

(11) **EP 2 999 392 B1**
(45) Date of publication and mention of the grant of the patent: **17.07.2019**
(21) Application number: 14801696.7
(22) Date of filing: 21.05.2014
(51) Int. Cl.: A61B 1/055, A61B 3/14, G06Q 50/22, G06T 7/00, A61B 1/00, A61B 3/10, A61B 5/00, G16H 40/67

(54) **SYSTEM AND METHOD FOR IMAGING A PATIENT USER'S BODY PART**
SYSTEM UND VERFAHREN ZUR ABBILDUNG EINES KÖRPERTEILS EINES PATIENTEN
SYSTÈME ET PROCÉDÉ POUR FAIRE L'IMAGERIE D'UNE PARTIE DU CORPS D'UN UTILISATEUR PATIENT

(30) Priority: 21.05.2013 US 201361825830 P
(43) Date of publication of application: 30.03.2016
(73) Proprietor: NeuroVision Imaging, Inc., Sacramento, California 95833 (US)
(72) Inventor: VERDOONER, Steven, Sacramento, CA 95833 (US)
(74) Representative: V.O.
(86) International application number: PCT/US2014/039034
(87) International publication number: WO 2014/190091

(56) References cited:
- US-A1- 2010 097 573
- US-A1- 2010 097 573
- US-A1- 2011 130 652
- US-A1- 2011 130 652
- US-B1- 6 485 413

## Description

### BACKGROUND OF THE INVENTION

### FIELD OF THE INVENTION:

The present invention is a system and method for imaging. More specifically, the present invention is a system and method for imaging a patient user's body part.

### DESCRIPTION OF THE RELATED ART:

Imaging of a patient user's body part is typically done with one or more slit lamps, ophthalmoscopes, fundus cameras, scanning laser ophthalmoscopes or SLO's and wide field devices for imaging the eye, an otoscope for imaging the ear, nose or throat, a dermascope for imaging the skin, or an endoscope for imaging an interior organ or body cavity. These devices are often relatively expensive and require one or more personal computers, cameras, sensors and monitors. They are typically relatively large, require an instrument table, are not portable, are not battery powered and require an experienced technician to operate. Even hand-held models of these devices are limited in their field of view. Typically these devices acquire a single image rather than a video stream. When retinal images are shot with different focus and alignment, it is often up to the observer to view multiple images to combine a composite in their mind if the images are in focus. While some of these devices allow control of focus, it is difficult to obtain a well-focused image throughout the depths of a three dimensional body part such as the retina. Additionally, there are optical aberrations that may be caused by the eye and/or imaging device that may cause regions to be out of focus. Alignment of the imaging device to a patient's eye also may affect overall clarity of specific image regions.

Otoscopes are typically hand-held devices that allow an observer to view an ear, a nose or a throat. Utilizing the components of a cell phone with an operating system or a SMARTPHONE® or a tablet computer, in combination with appropriate optics, allows for visualization, storage and transmission of images of an ear, just like images of an eye or a throat. Images may also be obtained of skin at visible or specific wavelengths for dermatological applications.

US20110130652 discloses an optical coherence tomography image that uses an interferometer to detect interference between reflections from a measurement point within an eye with reference light as a function of wavelength. The measurement point is scanned using a micro mechanical system to rotate a mirror. An image is formed from scanned columns. US20100097573 discloses an apparatus and method for imaging the eye that makes use of a slit lamp. The document mentions the possibility of creating a multi-focal plenoptic image, an image or movie that is created from images at multiple focal planes. An algorithm to combine images would automatically align the images while correcting for translation, rotation, curvature, and magnification differences between the images.

### BRIEF SUMMARY OF THE INVENTION

The present invention is a system and method for imaging. More specifically, the present invention is a system and method for imaging a patient user's body part.

The system and method for imaging a patient user's body part differs from other systems and methods in that traditional imaging devices do not afford for the visualization of multiple in-focus regions of the eye, retina, ear, nose, throat, skin or other interior or exterior body part, are not driven by SMARTPHONE® or tablet computer and are relatively large, expensive and cumbersome. The system and method solves this problem through a combination of packaging, optics and image registration in combination with image analysis and processing, to yield relatively high quality focused images, plenoptic images and movies. Additionally, by utilizing multiple images, overall resolution and image quality is greatly improved.

The system to image a patient user's body part may include a server system with a processor system, a communications interface, a communications system, an input system and an output system, the server system having access to a communications network, a memory system with an operating system, a communications module, a web browser module, a web server application and a patient user body part imaging non-transitory storage media and a website displaying a plurality of web pages residing on the patient user body part imaging non-transitory storage media.

The method for imaging a patient user's body part may include the steps of selecting an optical imaging device to image the patient user's body part, acquiring one or more data sets with the optical imaging device, registering the acquired data sets, performing image processing on the acquired data sets to identify as good data clear, well exposed portions of the data sets and eliminates poorly defined, one or more dark data sets or one or more aberrations that degrade imaging quality and recombining good data from the image processed data sets into a single image of the patient user's body part. The method may be executed by a non-transitory computer storage media having instructions stored thereon.

It is an object of the present invention to provide a system and method for imaging a patient user's body part where a data set is obtained from one or more existing devices.

It is an object of the present invention to provide a system and method for imaging a patient user's body part that are obtained from new devices specifically designed to create images that are in focus at various depths either through stepping focus or a multi-element microlens that is placed over a sensor that contains information from multiple image planes.

It is an object of the present invention to provide a system and method for imaging a patient user's body part that is applied to an optical coherence tomography or OCT data set to obtain a clear comprehensive OCT data set.

It is an object of the present invention to provide a system and method for imaging a patient user's body part where several interfaces are detachable for each imaging modality.

It is an object of the present invention to provide a system and method for imaging a patient user's body part that is non-mydriatic and may be switched between infra-red or IR and white light or other discreet spectral wavelength for utilization on patients without pharmacological dilation.

It is an object of the present invention to provide a system and method for imaging a patient user's body part that is vastly improved by recording movie streams, or rapidly acquired still images, and parsing good quality images and image sections from each image and combining them into single or multiple images and/or movies at relatively improved image quality.

It is an object of the present invention to provide a system and method for imaging a patient user's body part that is a trigger mechanism with electronics that interface with an electronic adapter on a SMARTPHONE® or a tablet computer.

It is an object of the present invention to provide a system and method for imaging a patient user's body part where a 60D lens, or similar large field retinal lens, is utilized to obtain a wide field image of a retina.

It is an object of the present invention to provide a system and method for imaging a patient user's body part where a stereo splitter is utilized to obtain 3-D images and information.

It is an object of the present invention to provide a system and method for imaging a patient user's body part where a high resolution image of a plurality of vessels is obtained to assess risk of stroke and cardiovascular events.

It is an object of the present invention to provide a system and method for imaging a patient user's body part where one or more retinal vessels are analyzed for tortuosity and detection of hypertension.

It is an object of the present invention to provide a system and method for imaging a patient user's body part where a device is utilized to detect Alzheimer's disease by one or more images of amyloid beta plaque in a retina.

It is an object of the present invention to provide a system and method for imaging a patient user's body part where a device is utilized to image, diagnose or screen for diabetic retinopathy, macular degeneration, glaucoma, cataracts or other ocular disorder.

It is an object of the present invention to provide a system and method for imaging a patient user's body part where a device is utilized to image the anterior segment of the eye for ophthalmic conditions.

It is an object of the present invention to provide a system and method for imaging a patient user's body part where an otoscope is utilized to diagnose ear, nose or throat infections.

It is an object of the present invention to provide a system and method for imaging a patient user's body part where a device is utilized to image skin for dermatological conditions.

It is an object of the present invention to provide a system and method for imaging a patient user's body part where a device is utilized to image dental conditions.

It is an object of the present invention to provide a system and method for imaging a patient user's body part where a device is utilized to image interior body organs or cavities for medical conditions.

It is an object of the present invention to provide a system and method for imaging a patient user's body part where a device is utilized for telemedicine applications.

It is an object of the present invention to provide a system and method for imaging a patient user's body part where the system controls are voice-activated.

It is an object of the present invention to provide a system and method for comparing plenoptic images taken at two or more different points in time by registering them with respect to each other, and playing the sequence as a movie.

It is an object of the present invention to provide a system and method for combining multiple overlapping plenoptic images to form a larger mosaicked image field covering the area of interest.

### BRIEF DESCRIPTION OF THE DRAWINGS

The present invention will be described by way of exemplary embodiments, but not limitations, illustrated in the accompanying drawing in which like references denote similar elements, and in which:
FIG. 1 illustrates a diagram of a single image, in accordance with one embodiment of the present invention.
FIG. 2 illustrates a diagram of a plurality of multiple images, in accordance with one embodiment of the present invention.
FIG. 3 illustrates a system overview of a system to image a patient user's body part, in accordance with one embodiment of the present invention.
FIG. 4 illustrates a block diagram of a server system, in accordance with one embodiment of the present invention.
FIG. 5 illustrates a block diagram of a client system, in accordance with one embodiment of the present invention.
FIG. 6 illustrates a flowchart of a method for imaging a patient user's body part, in accordance with one embodiment of the present invention.
FIG. 7 illustrates a side view of a removable lens, in accordance with one embodiment of the present invention.

### DETAILED DESCRIPTION OF ILLUSTRATIVE EMBODIMENTS

Various aspects of the illustrative embodiments will be described using terms commonly employed by those skilled in the art to convey the substance of their work to others skilled in the art. However, it will be apparent to those skilled in the art that the present invention may be practiced with only some of the described aspects. For purposes of explanation, specific numbers, materials and configurations are set forth in order to provide a thorough understanding of the illustrative embodiments. However, it will be apparent to one skilled in the art that the present invention may be practiced without the specific details. In other instances, well-known features are omitted or simplified in order not to obscure the illustrative embodiments.

Various operations will be described as multiple discrete operations, in turn, in a manner that is most helpful in understanding the present invention. However, the order of description should not be construed as to imply that these operations are necessarily order dependent. In particular, these operations need not be performed in the order of presentation.

The phrase "in one embodiment" is used repeatedly. The phrase generally does not refer to the same embodiment, however, it may. The terms "comprising", "having" and "including" are synonymous, unless the context dictates otherwise.

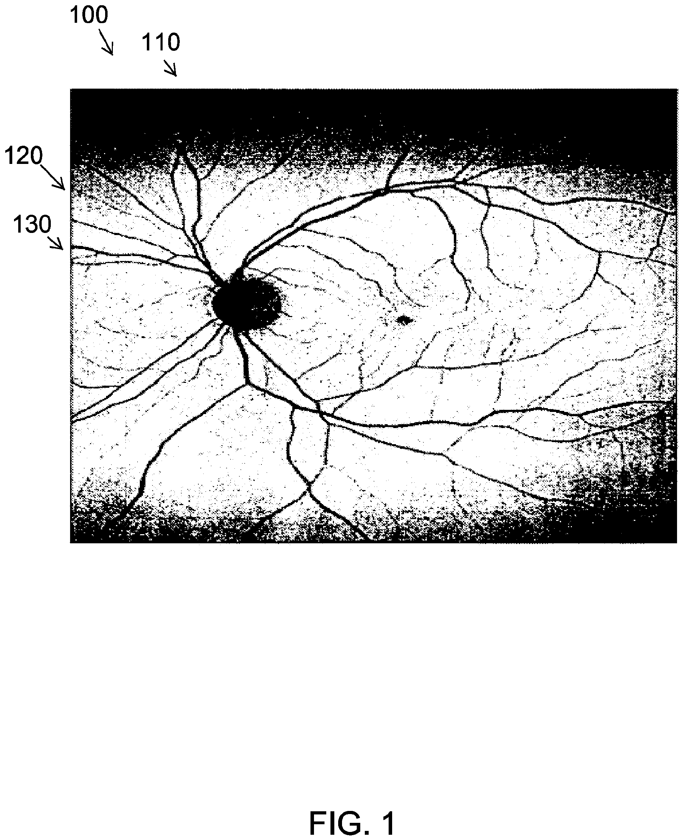

FIG. 1 illustrates a diagram of a single image 100, in accordance with one embodiment of the present invention.

The single image 100 may be of a patient user's body part 110. More specifically, the single image 100 may include amyloid beta plaque and drusen 120 or one or more retinal vessels 130 disposed on the patient user's body part 110. The patient user's body part 110 may be a retina, an eye, a nose, a throat or skin or other suitable patient user's body part.

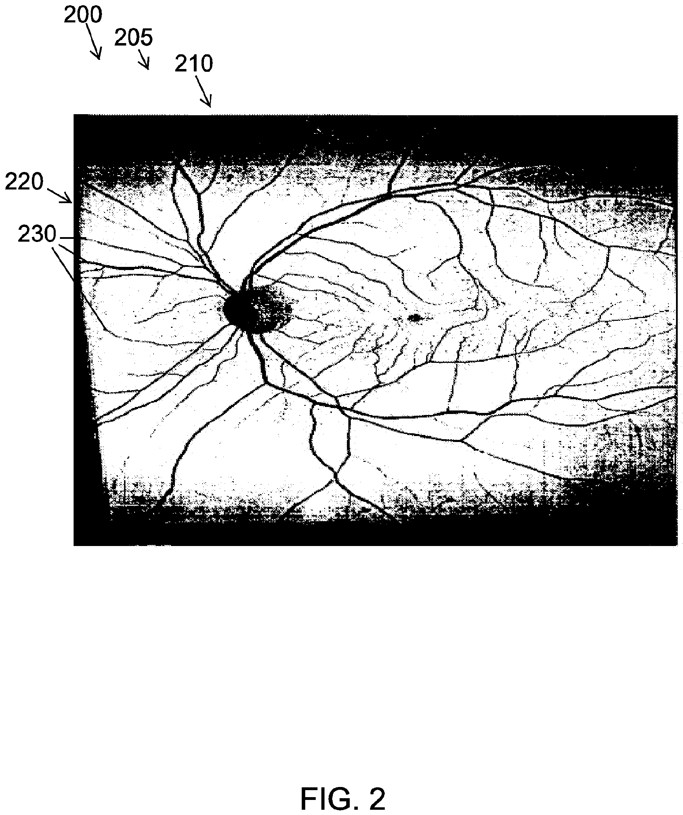

FIG. 2 illustrates a diagram of a plurality of multiple images 200, in accordance with one embodiment of the present invention.

The multiple images 200 may be registered and combined into a single well-focused image 205. The multiple images 200 may be of a patient user's body part 210. More specifically, the multiple images 200 may include amyloid beta plaque and drusen 220 or one or more retinal vessels 230 disposed on the patient user's body part 210. The patient user's body part 210 may be an eye, a nose, a throat or skin or other suitable patient user's body part. The multiple images 200 may have improved resolution, focus, dynamic range and image quality than the single image 100 illustrated in FIG. 1.

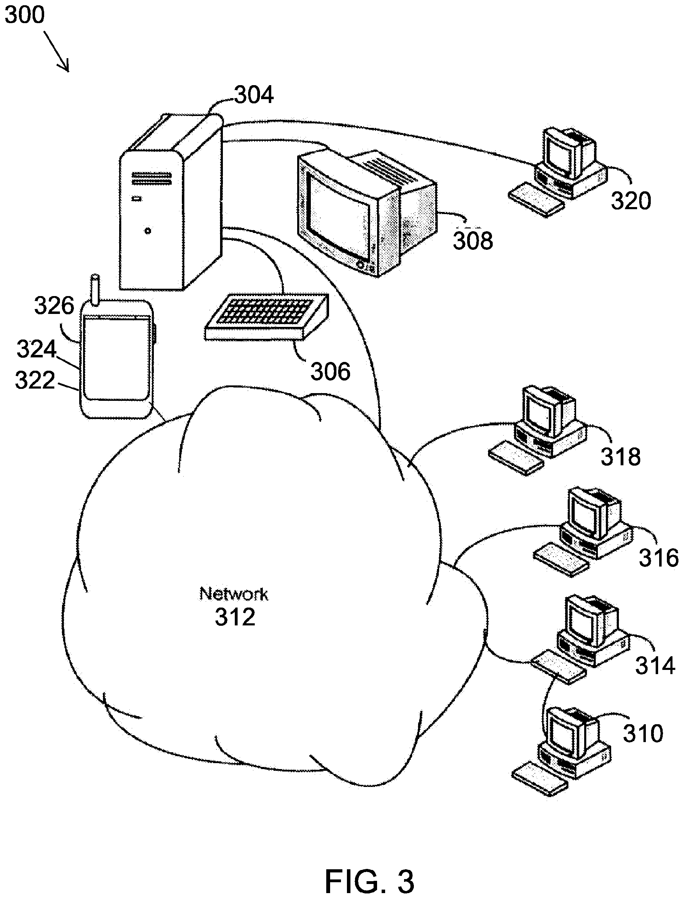

FIG. 3 illustrates a system overview of a system 300 to image a patient user's body part, in accordance with one embodiment of the present invention.

The system 300 may include a server system 304, an input system 306, an output system 308, a plurality of client systems 310, 314, 316, 318 and 320, a communications network 312 and a handheld or mobile device 322. In other embodiments, the system 300 may include additional components and/or may not include all of the components listed above.

The server system 304 may include one or more servers. One server 304 may be the property of the distributor of any related software or non-transitory storage media. In other embodiments, the system 300 may include additional components and/or may not include all of the components listed above.

The input system 306 may be utilized for entering input into the server system 304, and may include any one of, some of, any combination of, or all of a keyboard system, a mouse system, a track ball system, a track pad system, a plurality of buttons on a handheld system, a mobile system, a scanner system, a wireless receiver, a microphone system, a connection to a sound system, and/or a connection and/or an interface system to a computer system, an intranet, and/or the Internet (i.e., IrDA, USB).

The output system 308 may be utilized for receiving output from the server system 304, and may include any one of, some of, any combination of or all of a monitor system, a wireless transmitter, a handheld display system, a mobile display system, a printer system, a speaker system, a connection or an interface system to a sound system, an interface system to one or more peripheral devices and/or a connection and/or an interface system to a computer system, an intranet, and/or the Internet.

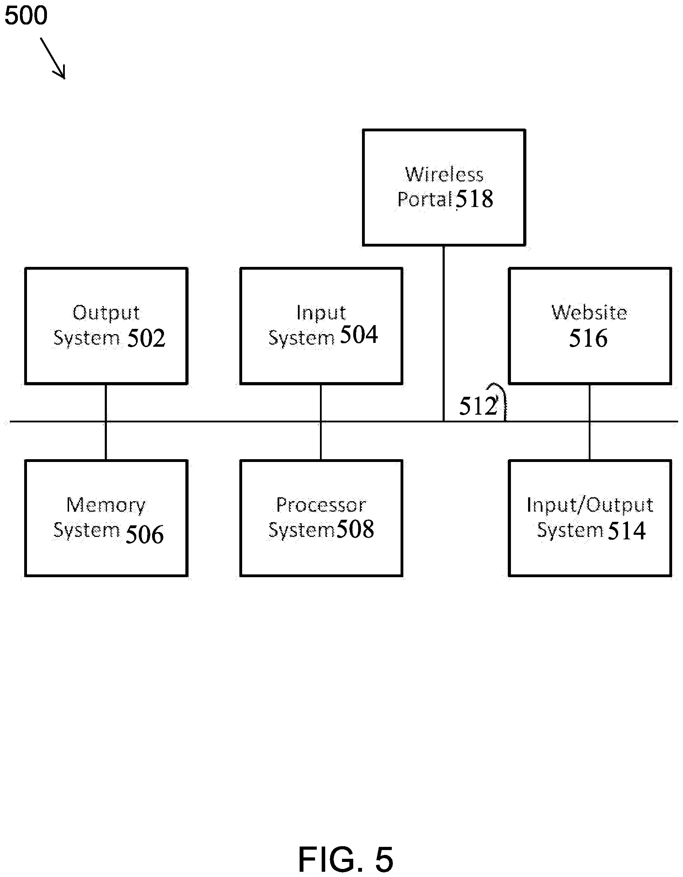

The system 300 may illustrate some of the variations of the manners of connecting to the server system 304, which may be a website (FIG. 5, 516) such as an information providing website (not shown). The server system 304 may be directly connected and/or wirelessly connected to the plurality of client systems 310, 314, 316, 318 and 320 and may be connected via the communications network 312. Client systems 320 may be connected to the server system 304 via the client system 318. The communications network 312 may be any one of, or any combination of, one or more local area networks or LANs, wide area networks or WANs, wireless networks, telephone networks, the Internet and/or other networks. The communications network 312 may include one or more wireless portals. The client systems 310, 314, 316, 318 and 320 may be any system that an end user may utilize to access the server system 304. For example, the client systems 310, 314, 316, 318 and 320 may be personal computers, workstations, tablet computers, laptop computers, game consoles, hand-held network enabled audio/video players, mobile devices and/or any other network appliance.

The client system 320 may access the server system 304 via the combination of the communications network 312 and another system, which may be the client system 318. The client system 320 may be a handheld or mobile wireless device 322, such as a mobile phone, a tablet computer or a handheld network-enabled audio/music player, which may also be utilized for accessing network content. The client system 320 may be a cell phone with an operating system or SMARTPHONE® 324 or a tablet computer with an operating system or IPAD® 326.

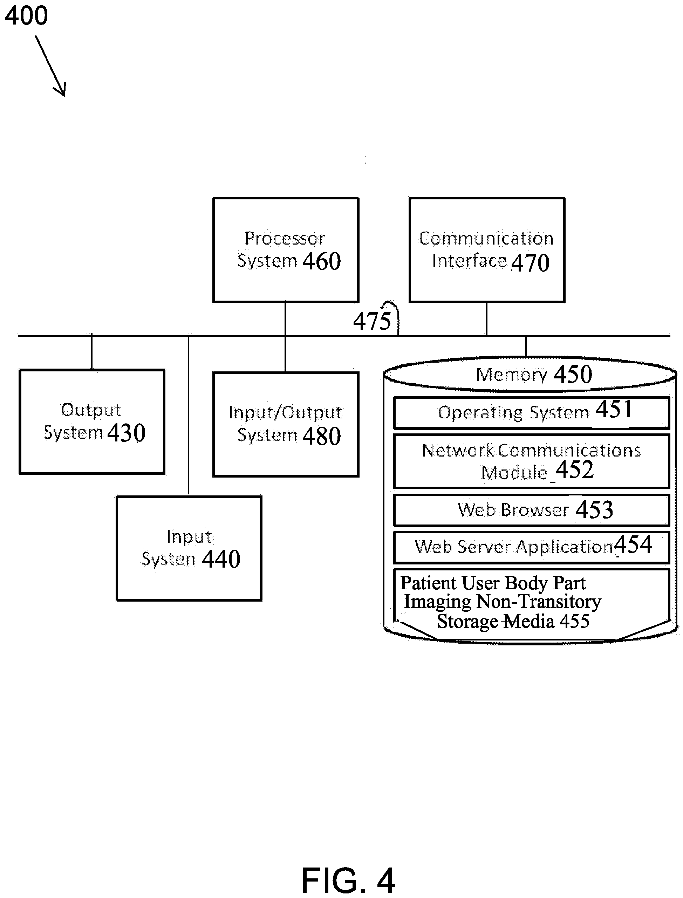

FIG. 4 illustrates a block diagram of a server system 400, in accordance with one embodiment of the present invention.

The server system 400 may include an output system 430, an input system 440, a memory system 450, which may store an operating system 451, a communications module 452, a web browser module 453, a web server application 454 and a patient user body part imaging non-transitory storage media 455. The server system 400 may also include a processor system 460, a communications interface 470, a communications system 475 and an input/output system 480. In other embodiments, the server system 400 may include additional components and/or may not include all of the components listed above.

The output system 430 may include any one of, some of, any combination of, or all of a monitor system, a handheld display system, a printer system, a speaker system, a connection or interface system to a sound system, an interface system to one or more peripheral devices and/or a connection and/or interface system to a computer system, an intranet, and/or the Internet.

The input system 440 may include any one of, some of, any combination of, or all of a keyboard system, a mouse system, a track ball system, a track pad system, one or more buttons on a handheld system, a scanner system, a microphone system, a connection to a sound system, and/or a connection and/or an interface system to a computer system, an intranet, and/or the Internet (i.e., IrDA, USB).

The memory system 450 may include any one of, some of, any combination of, or all of a long term storage system, such as a hard drive; a short term storage system, such as a random access memory; or a removable storage system, such as a floppy drive or a removable drive and/or a flash memory. The memory system 450 may include one or more machine readable mediums that may store a variety of different types of information. The term machine readable medium may be utilized to refer to any medium capable of carrying information that may be readable by a machine. One example of a machine-readable medium may be a computer-readable medium such as a non-transitory storage media. The memory system 450 may store one or more machine instructions for imaging a patient user's body part. The operating system 451 may control all software or non-transitory storage media and hardware of the system 100. The communications module 452 may enable the server system 304 to communicate on the communications network 312. The web browser module 453 may allow for browsing the Internet. The web server application 454 may serve a plurality of web pages to client systems that request the web pages, thereby facilitating browsing on the Internet.

The processor system 460 may include any one of, some of, any combination of, or all of multiple parallel processors, a single processor, a system of processors having one or more central processors and/or one or more specialized processors dedicated to specific tasks. The processor system 460 may implement the machine instructions stored in the memory system 450.

In an alternative embodiment, the communication interface 470 may allow the server system 400 to interface with the network 312. In this embodiment, the output system 430 may send communications to the communication interface 470. The communications system 475 communicatively links the output system 430, the input system 440, the memory system 450, the processor system 460 and/or the input/output system 480 to each other. The communications system 475 may include any one of, some of, any combination of, or all of one or more electrical cables, fiber optic cables, and/or sending signals through air or water (i.e., wireless communications). Some examples of sending signals through air and/or water may include systems for transmitting electromagnetic waves such as infrared and/or radio waves and/or systems for sending sound waves.

The input/output system 480 may include devices that have the dual function as the input and output devices. For example, the input/output system 480 may include one or more touch sensitive screens, which display an image and therefore may be an output device and accept input when the screens may be pressed by a finger or a stylus. The touch sensitive screens may be sensitive to heat and/or pressure. One or more of the input/output devices may be sensitive to a voltage or a current produced by a stylus. The input/output system 480 may be optional and may be utilized in addition to or in place of the output system 430 and/or the input device 440.

FIG. 5 illustrates a block diagram of a client system 500, in accordance with one embodiment of the present invention.

The client system 500 may include an output system 502, an input system 504, a memory system 506, a processor system 508, a communications system 512, an input/output system 514, a website 516 and a wireless portal 518. Other embodiments of the client system 500 may not have all of the components and/or may have other embodiments in addition to or instead of the components listed above.

The client system 500 may be any one of the client systems 310, 314, 316, 318, 320 and/or handheld or mobile wireless device 322, SMARTPHONE® 324 or IPAD® 326 that may be utilized as one of the network devices of FIG. 3. In other embodiments, the client system 500 may include additional components and/or may not include all of the components listed above. The output system 502 may include any one of, some of, any combination of or all of a monitor system, a wireless transmitter, a handheld display system, a printer system, a speaker system, a connection or interface system to a sound system, an interface system to peripheral devices and/or a connection and/or an interface system to a computer system, an intranet, and/or the Internet.

The input system 504 may include any one of, some of, any combination of or all of a keyboard system, a mouse system, a track ball system, a track pad system, one or more buttons on a handheld system, a scanner system, a wireless receiver, a microphone system, a connection to a sound system, and/or a connection and/or an interface system to a computer system, an intranet, and/or the Internet (i.e., Infrared Data Association or IrDA, Universal Serial Bus or USB).

The memory system 506 may include, any one of, some of, any combination of or all of a long-term storage system, such as a hard drive, a short term storage system, such as a random access memory; a removable storage system, such as a floppy drive or a removable drive and/or a flash memory. The memory system 506 may include one or more machine readable mediums that may store a variety of different types of information. The term machine readable medium may be utilized to refer to any medium that may be structurally configured for carrying information in a format that may be readable by a machine. One example of a machine-readable medium may be a computer-readable medium. The memory system 506 may store a non-transitory storage media for imaging a patient user body part.

The processor system 508 may include any one of, some of, any combination of, or all of multiple parallel processors, a single processor, a system of processors having one or more central processors and/or one or more specialized processors dedicated to specific tasks. The processor system 508 may implement the programs stored in the memory system 506. The communications system 512 may communicatively link the output system 502, the input system 504, the memory system 506, the processor system 508, and/or the input/output system 514 to each other. The communications system 512 may include any one of, some of, any combination of, or all of one or more electrical cables, fiber optic cables, and/or means of sending signals through air or water (i.e., wireless communications). Some examples of means of sending signals through air and/or water may include systems for transmitting electromagnetic waves such as infrared and/or radio waves and/or systems for sending sound waves.

The input/output system 514 may include devices that have the dual function as input and output devices. For example, the input/output system 514 may include one or more touch sensitive screens, which display an image and therefore may be an output device and accept input when the screens may be pressed by a finger or a stylus. The touch sensitive screens may be sensitive to heat, capacitance and/or pressure. One or more of the input/output devices may be sensitive to a voltage or a current produced by a stylus. The input/output system 514 is optional, and may be utilized in addition to or in place of the output system 502 and/or the input device 504.

The client systems 310, 314, 316, 318, 320 and the handheld wireless device 322 may also be tied into a website 516 or a wireless portal 518 which may also be tied directly into the communications system 512. Any website 516 or wireless portal 518 may also include a non-transitory storage media and a website module (not shown) to maintain, allow access to and run the website as well.

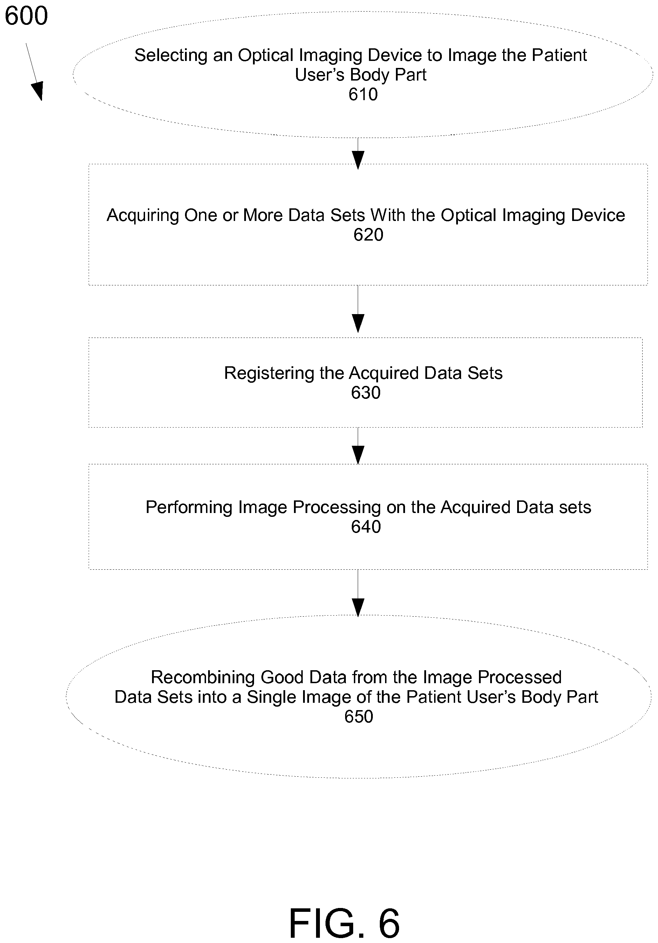

FIG. 6 illustrates a flowchart of a method 600 for imaging a patient user's body part, in accordance with one embodiment of the present invention.

The method 600 may include the steps of selecting an optical imaging device to image the patient user's body part 610, acquiring one or more data sets with the optical imaging device 620, registering the acquired data sets 630, performing image processing on the acquired data sets 640 and recombining good data from the image processed data sets into a single image of the patient user's body part 650.

The selecting step 610 may include the optical imaging device is selected from the group consisting of a slit lamp mounted device, a slit lamp integrated device, an optical coherence tomography or OCT device, an optical imaging at one or more specific wavelengths device, a multispectral device, a hyper spectral device, an autofluorescence device, a confocal retinal imaging device, a scanning laser ophthalmoscope device, an adaptive optics device, a polarization orientation specific device, a fundus camera, a handheld imager device, a direct ophthalmoscope, an indirect ophthalmoscope, a fluorescein angiography device, an ICG angiography device, a curcumin fluorescence imaging auto-fluorescence imaging device, an otoscope, a dermascope, or an endoscope. The acquiring step 620 may be performed with continuous thru-focus and exposure control or deliberate focus and exposure control. The registering step 630 may include the data sets are automatically registered with sub pixel accuracy. The performing step 640 may identify clear, well exposed portions of the data sets and eliminates poorly defined, one or more dark data sets or one or more aberrations that degrade imaging quality. The recombining step 650 may include the single image is plenoptic or in focus at multiple depths. The recombining step 650 may include a movie file is created that allows step through a focus stack or select a region wanted to view that is in focus.

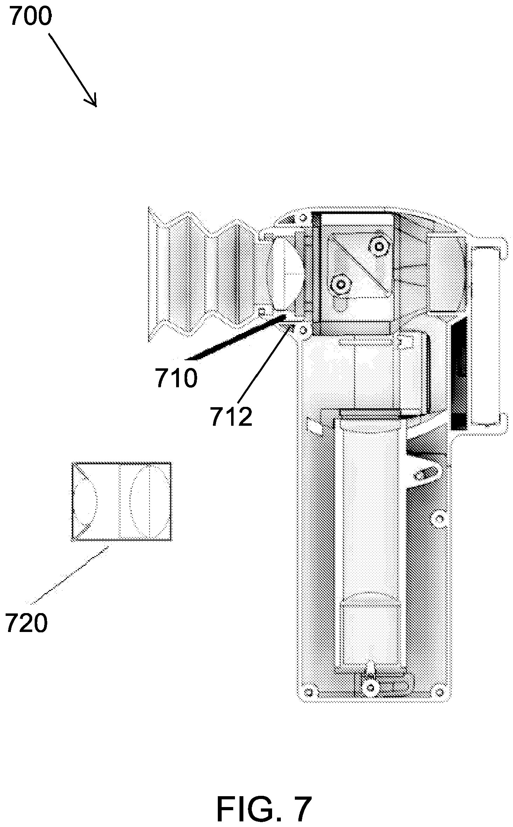

FIG. 7 illustrates a side view of a removable lens 700, in accordance with one embodiment of the present invention.

The removable lens 700 may include a macro lens 710 and an exchangeable lens assembly 720. The macro lens 710 may also be a derm lens 712 or other suitable type of lens. The exchangeable lens assembly 720 may be coupled to the system (FIG. 3, 300). The exchangeable lens assembly 720 may receive the macro lens 710, thereby coupling the macro lens 710 to the exchangeable lens assembly 720. The removable lens 700 may also be swapped with other removable lens 700.

The system and method may be utilized alone or in combination with another device for a variety of patient user's body part imaging modalities. More specific to eye indications, the system and method may be utilized on but not limited to slit lamp mounted, slit lamp integrated, OCT, optical imaging at specific wavelengths, multispectral, hyper spectral, autofluorescence, confocal retinal imaging, scanning laser ophthalmoscope, adaptive optics, polarization orientation specific, fundus cameras, hand-held imagers, direct and indirect ophthalmoscopes, fluorescein angiography, Indocyanine green or ICG angiography, curcumin fluorescence imaging autofluorescence, otoscope, derma scopes and other imaging modalities. Data sets may be acquired either with random or deliberate focus and exposure control. Data sets may also be obtained using specified illumination control which is linked in mode/time to an external focusing or illumination device. Data sets may be automatically registered with sub-pixel accuracy. Image processing may be performed on data sets to identify clear, well-exposed portions of data sets and eliminating poorly defined and/or dark data sets or other aberrations that degrade imaging quality. Good data may be recombined into a single image that is plenoptic or in focus at multiple depths and/or a movie file may be created that allows a user to step through a focus stack or select a region that they want to view that is in focus.

The system and method may be utilized with a variety of SMARTPHONES® and tablet computers that incorporate camera, display, computing power and communication into a single package. The system and method may be utilized with but not limited to PHONE®, iPAD®, ANDROID™ phones and tablet computers, WINDOWS™ phones and tablet computers, or other portable devices. The system and method may be utilized with or without discreet focus control. The system and method may be applied across a number of eye imaging modalities including but not limited to color fundus imaging, anterior segment imaging, cornea and lens imaging, fluorescein angiography, ICG angiography, curcumin fluorescence imaging, autofluorescence, discreet wavelength imaging, red-free, hyper and multispectral imaging and optical coherence tomography. Each of these modalities allows for registration of image data sets and subsequent image processing to obtain relatively high-frequency in-focus, well exposed regions from each image, combined into a single image or a plenoptic multi-focal single image, or movie image that allows the user to step through or select regions to be viewed that are in focus. Images may be selected manually or automatically to aggregate into a high resolution panoramic image and/or multiple images may be registered and combined into a single image to greatly improve image quality. In order to achieve artifact-free images, the system and method utilizes a light emitting diode or LED light source (or other light source, or a light source built into a camera or tablet computer) that may be off-axis from the central optical imaging path. A flipper arm may be introduced to momentarily block the artifact and thereby render images that mask the central artifact. Physical or electronic illumination control devices such as DMD arrays may be employed for illumination control. Images with and without flipper may be recombined to create an artifact-free image. Elimination of central artifact may also be accomplished by oscillating an optic, optical component or patient fixation to create image sets that have artifacts in different geographic locations. Images may then be combined with or without flipper to obtain artifact free images. The system and method may also utilize a disposable eye cup to create a darkened environment and be sanitary. The system and method may have a dedicated set of optics and interface so as to be utilized as an otoscope to image an ear, a nose, a throat or skin. Images may be stored, reviewed or sent for telemedicine consultation. The dedicated optics may be detachable in part to allow easy switching between modalities.

The system and method may be utilized to image an eye, an ear, a nose, a throat and skin for documentation of anatomy and/or detection of pathology. More specifically for the eye, the system and method may be utilized for both imaging of an anterior segment and a posterior segment of an eye and also for substructure as seen on OCT. One aspect of the system and method may be the automated registration of images and then subsequent image processing to identify regions that are relatively well-focused and evenly illuminated and to extract high frequency information like a Weiner filter, then recombining them into a single image. An algorithm may also be capable of eliminating areas of images that are poorly focused, that contain other optical aberrations and/or are not well illuminated. The system and method may be applied to a new SMARTPHONE®, a tablet computer and other patient user's body part imaging devices that specifically step the focus and/or existing devices that may or may not require the user to change the focus. The system and method may also be applied by deliberately stepping the focus of a device to generate an image set.

The system may also register current images with one or more previously captured and processed images to allow direct comparison of feature changes over time. The sequence of images over time may be presented either side-by-side, or as a sequence played as a movie that repeats over time, with user controlled frame rate.

Information from previously collected datasets may be used to guide the collection of new images to ensure they are of the same feature area. This may involve a visual feedback mechanism presented to the user, such as an image overlaid on real-time video of the area to be imaged.

While the present invention has been related in terms of the foregoing embodiments, those skilled in the art will recognize that the present invention is not limited to the embodiments described. The present invention is defined in the appended claims.

## Claims

1. A system to image a patient user body part, comprising:
a server system (400) with a processor system (460), a communications interface (470), a communications system (475), an input system (440) and an output system (430), the server system (400) having access to a communications network (312);
a memory system (450) with an operating system (451), a communications module (452), a web browser module (453), a web server application (454) and a patient user body part imaging non-transitory storage media (455); and
a website (510) displaying a plurality of web pages residing on the patient user body part imaging non-transitory storage media,
wherein the patient user body part imaging non-transitory storage media is configured to acquire (620) a plurality of data sets with an optical imaging device, (630) wherein the system to image apatient is configured to register the acquired data sets, to perform (640) image processing on the acquired data sets to identify as good data clear, well exposed portions of the data sets and eliminates poorly defined, one or more dark data sets or one or more aberrations that degrade imaging quality; and to recombine (650) good data from the image processed data sets into a single image of the patient user's body part.

2. The system to image a patient user body part according to claim 1, further comprising a client system (500), wherein the client system includes an output system (502), an input system (504), a memory system (506), a processor system (508) and a communications system (512).

3. The system to image a patient user body part according to claim 1, further comprising a removable lens (700).

4. The system to image a patient user body part according to claim 3, wherein the removable lens (700) includes a macro lens (710) and an exchangeable lens assembly (720), the exchangeable lens assembly (720) receives the macro lens (710), thereby coupling the macro lens (710) to the exchangeable lens assembly (720).

5. A method for imaging a patient user body part, comprising the steps of:
selecting (610) an optical imaging device to image the patient user's body part;
acquiring (620) a plurality of data sets with the optical imaging device, the acquiring is performed with focus at one or more axial positions and exposure control or deliberate focus at one or more specified image locations and exposure control;
registering (630) the acquired data sets;
performing (640) image processing on the acquired data sets to identify as good data clear, well exposed portions of the data sets and eliminates poorly defined, one or more dark data sets or one or more aberrations that degrade imaging quality; and
recombining (650) good data from the image) processed data sets into a single image of the patient user's body part.

6. The method according to claim 5, wherein the optical imaging device is selected from the group consisting of a slit lamp mounted device, a slit lamp integrated device, an OCT device, an optical imaging at one or more specific wavelengths device, a multispectral device, a hyper spectral device, an Autofluorescence device, a confocal retinal imaging device, a scanning laser ophthalmoscope device, an adaptive optics device, a polarization orientation specific device, a fundus camera, a handheld imager device, a direct ophthalmoscope, an indirect ophthalmoscope, a fluorescein angiography device, an ICG angiography device, a curcumin fluorescence imaging autofluorescence imaging device, an otoscope or a dermascope, or endoscope.

7. The method according to claim 5, wherein the data sets are automatically registered with sub pixel accuracy.

8. The method according to claim 5, wherein the image frames are registered to compensate for translation, rotation, perspective shift, and subframe warping.

9. The method according to claim 5, wherein the image processing on the acquired datasets involves image enhancement such as Wienor filtering or iterative deconvolution,

10. The method according to claim 5, wherein multiple overlapping plenoptic images are mosaicked together to form a larger image field covering the area of interest.

11. The method according to claim 5, wherein the current image is registered with one or more previously captured and processed images to allow direct comparison of feature changes over time.

12. The method according to claim 5, wherein the single image is plenoptic or in focus at multiple depths and a movie file is created that allows the user to step through a focus stack or select a region to view that is in focus.

13. A non-transitory computer storage media having instructions stored thereon which, when executed, execute a method comprising the steps of any one of claims 5-12.

## Patentansprüche

1. System zur Abbildung eines Körperteils eines Patienten-Anwenders, umfassend:
ein Serversystem (400) mit einem Prozessorsystem (460), einer Kommunikationsschnittstelle (470), einem Kommunikationssystem (475), einem Eingabesystem (440) und einem Ausgabesystem (430), wobei das Serversystem (400) Zugang zu einem Kommunikationsnetzwerk (312) hat;
ein Speichersystem (450) mit einem Betriebssystem (451), einem Kommunikationsmodul (452), einem Webbrowsermodul (453), einer Webserveranwendung (454) und einem nicht transienten Speichermedium (455) für Körperteilabbildungen eines Patienten-Anwenders; und
eine Website (516), die eine Vielzahl von Webseiten anzeigt, basierend auf dem Speichermedium für Körperteilabbildungen des Patienten-Anwenders, wobei das nicht transiente Speichermedium für Körperteilabbildungen des Patienten-Anwenders so konfiguriert ist, dass es mehrere Datensätze mit einer optischen Bildgebungsvorrichtung (630) erfasst (620), wobei das System zum Abbilden eines Patienten dazu konfiguriert ist, die erfassten Datensätze zu registrieren, eine Bildverarbeitung an den erfassten Datensätzen durchzuführen (640), um klare, gut belichtete Teile der Datensätze als gute Daten zu identifizieren und schlecht definierte, einen oder mehrere dunkle Datensätze oder eine oder mehrere Aberrationen, die die Abbildungsqualität beeinträchtigen, zu beseitigen; und (650) gute Daten aus den bildverarbeiteten Datensätzen zu einer einzigen Abbildung des Körperteils des Patienten-Anwenders zu rekombinieren.

2. System zur Abbildung eines Körperteils eines Patienten-Anwenders nach Anspruch 1, ferner umfassend ein Client-System (500), wobei das Client-System ein Ausgabesystem (502), ein Eingabesystem (504), ein Speichersystem (506), ein Prozessorsystem (508) und ein Kommunikationssystem (512) umfasst.

3. System zum Abbilden eines Körperteils eines Patienten-Anwenders nach Anspruch 1, ferner umfassend eine herausnehmbare Linse (700).

4. System zur Abbildung eines Körperteils eines Patienten-Anwenders nach Anspruch 3, wobei die herausnehmbare Linse (700) eine Makrolinse (710) und eine austauschbare Linsenbaugruppe (720) umfasst, wobei die austauschbare Linsenbaugruppe (720) die Makrolinse (710) aufnimmt, wodurch die Makrolinse (710) mit der austauschbaren Linsenbaugruppe (720) gekoppelt wird.

5. Verfahren zum Abbilden eines Körperteils eines Patienten-Anwenders, umfassend die Schritte:
Auswählen (610) einer optischen Bildgebungsvorrichtung, um den Körperteil des Patienten-Anwenders abzubilden;
Erfassen (620) mehrerer Datensätze mit der optischen Bildgebungsvorrichtung, wobei das Erfassen mit Fokus an einer oder mehreren axialen Positionen und Belichtungssteuerung oder bewusstem Fokus an einem oder mehreren bestimmten Abbildungsorten und Belichtungssteuerung durchgeführt wird;
Registrieren (630) der erfassten Datensätze;
Durchführen (640) der Bildverarbeitung auf den erfassten Datensätzen, um klare, gut belichtete Teile der Datensätze als gute Daten zu identifizieren, und als schlecht definierte, einen oder mehrere dunkle Datensätze oder eine oder mehrere Aberrationen, die die Abbildungsqualität beeinträchtigen, zu beseitigen;
und
Rekombinieren (650) von guten Daten aus den bildverarbeiteten Datensätzen zu einer einzigen Abbildung des Körperteils des Patienten-Anwenders.

6. Verfahren nach Anspruch 5, wobei die optische Bildgebungsvorrichtung aus der Gruppe ausgewählt ist, die aus einer an einer Spaltlampe angebrachten Vorrichtung, einer in einer Spaltlampe integrierten Vorrichtung, einer OCT-Vorrichtung, einer optischen Bildgebungsvorrichtung mit einer oder mehreren spezifischen Wellenlängen, einer multispektralen Vorrichtung, einer hyperspektralen Vorrichtung, einer Autofluoreszenzvorrichtung, einer konfokalen retinalen Bildgebungsvorrichtung, einer Abtastlaser-Ophthalmoskopvorrichtung, einer adaptiven Optikvorrichtung, einer speziellen polarisationsorientierten Vorrichtung, einer Funduskamera, einer tragbaren Bildgebungsvorrichtung, einem direkten Ophthalmoskop, einem indirekten Ophthalmoskop, einem Fluoreszenzangiographiegerät, einem ICG-Angiographiegerät, einer Autofluoreszenz-Bildgebungsvorrichtung mit Curcumin-Fluoreszenzbildgebung, einem Otoskop oder einem Dermaskop oder Endoskop, besteht.

7. Verfahren nach Anspruch 5, wobei die Datensätze automatisch mit Subpixelgenauigkeit registriert werden.

8. Verfahren nach Anspruch 5, wobei die Bildframes registriert sind, um Verschiebung, Drehung, Perspektivverschiebung und Subframe-Verzug zu kompensieren.

9. Verfahren nach Anspruch 5, wobei die Bildverarbeitung in den erfassten Datensätzen eine Bildverbesserung wie etwa eine Wiener-Filterung oder eine iterative Dekonvolution beinhaltet.

10. Verfahren nach Anspruch 5, bei dem mehrere überlappende plenoptische Bilder miteinander mosaikiert werden, um ein größeres Bildfeld zu bilden, das den interessierenden Bereich abdeckt.

11. Verfahren nach Anspruch 5, wobei das aktuelle Bild mit einem oder mehreren zuvor erfassten und verarbeiteten Bildern registriert wird, um einen direkten Vergleich von Merkmalsänderungen über die Zeit zu ermöglichen.

12. Verfahren nach Anspruch 5, wobei das einzelne Bild in mehreren Tiefen plenoptisch oder fokussiert ist und eine Filmkachel erstellt wird, die es dem Benutzer ermöglicht, einen Fokusstapel zu durchlaufen oder einen Bereich auszuwählen, um das Objekt im Fokus anzuzeigen.

13. Nicht transientes Computerspeichermedium, auf dem Anweisungen gespeichert sind, die bei Ausführung ein Verfahren ausführen, das die Schritte nach einem der Ansprüche 5-12 umfasst.

## Revendications

1. Système d'imagerie d'une partie de corps de patient utilisateur, comprenant :
un système de serveur (400) avec un système de processeur (460), une interface de communication (470), un système de communication (475), un système d'entrée (440) et un système de sortie (430), le système de serveur (400) ayant accès à un réseau de communication (312) ;
un système de mémoire (450) avec un système d'exploitation (451), un module de communication (452), un module de navigateur web (453), une application de serveur web (454) et un support de stockage non transitoire d'imagerie de partie de corps de patient utilisateur (455) ; et
un site web (516) affichant une pluralité de pages web résidant sur le support de stockage non transitoire d'imagerie de partie de corps de patient utilisateur,
le support de stockage non transitoire d'imagerie de partie de corps de patient utilisateur étant configuré pour acquérir (620) une pluralité d'ensembles de données avec un dispositif d'imagerie optique (630)
le système d'imagerie d'un patient étant configuré pour aligner les ensembles de données acquis, pour effectuer (640) un traitement d'image sur les ensembles de données acquis pour identifier en tant que données claires satisfaisantes, des parties bien exposées des ensembles de données et élimine un ou plusieurs ensembles de données sombres mal définis ou une ou plusieurs aberrations qui dégradent la qualité d'imagerie ;
et pour recombiner (650) les données satisfaisantes des ensembles de données d'image traités dans une image unique de la partie de corps de patient utilisateur.

2. Système d'imagerie d'une partie de corps de patient utilisateur selon la revendication 1, comprenant en outre un système client (500), le système client comprenant un système de sortie (502), un système d'entrée (504), un système de mémoire (506), un système de processeur (503) et un système de communication (512).

3. Système d'imagerie d'une partie de corps de patient utilisateur selon la revendication 1, comprenant en outre un objectif amovible (700).

4. Système d'imagerie d'une partie de corps de patient utilisateur selon la revendication 3, dans lequel l'objectif amovible (700) comprend un objectif macro (710) et un ensemble d'objectif échangeable (720), l'ensemble d'objectif échangeable (720) reçoit l'objectif macro (710), de façon à coupler dans celui-ci l'objectif macro (710) à l'ensemble d'objectif échangeable (720).

5. Procédé d'imagerie d'une partie de corps de patient utilisateur, comprenant les étapes de :
sélection (610) d'un dispositif d'imagerie optique pour imager la partie de corps du patient utilisateur ;
acquisition (620) d'une pluralité d'ensembles de données avec le dispositif d'imagerie optique, l'acquisition étant effectuée avec une mise au point à une ou plusieurs positions axiales et une commande d'exposition ou une mise au point délibérée à un ou plusieurs emplacements d'image et une commande d'exposition spécifiés ;
alignement (630) des ensembles de données acquis ;
la conduite (640) d'un traitement d'image sur les ensembles de données acquis pour identifier en tant que données claires satisfaisantes, des parties bien exposées des ensembles de données et élimine un ou plusieurs ensembles de données sombres mal définis ou une ou plusieurs aberrations qui dégradent la qualité d'imagerie ;
et
recombinaison (650) des données satisfaisantes des ensembles de données d'image traités dans une image unique de la partie de corps de patient utilisateur.

6. Procédé selon la revendication 5, dans lequel le dispositif d'imagerie optique est choisi dans le groupe constitué d'un dispositif monté à lampe à fente, un dispositif intégré à lampe à fente, un dispositif OCT, un dispositif d'imagerie optique à une ou plusieurs longueurs d'onde spécifiques, un dispositif multispectral, un dispositif hyperspectral, un dispositif à autofluorescence, un dispositif d'imagerie rétinienne confocale, un dispositif d'ophtalmoscope laser à balayage, un dispositif optique adaptatif, un dispositif spécifique à orientation de polarisation, un rétinographe, un dispositif d'imagerie portatif, un ophtalmoscope direct, un ophtalmoscope indirect, un dispositif d'angiographie à la fluorescéine, un dispositif d'angiographie ICG, un dispositif d'imagerie par autofluorescence d'imagerie de fluorescence de curcumine, un otoscope ou un dermascope, ou un endoscope.

7. Procédé selon la revendication 5, dans lequel les ensembles de données sont automatiquement alignés avec une précision sous-pixel.

8. Procédé selon revendication 5, dans lequel les trames d'image sont alignées pour compenser la translation, la rotation, le décalage de perspective et le gauchissement sous-trame.

9. Procédé selon la revendication 5, dans lequel le traitement d'image sur les ensembles de données acquis met en oeuvre une accentuation d'image telle qu'un filtrage de Wiener ou une déconvolution itérative.

10. Procédé selon la revendication 5, dans lequel des images plénoptiques superposées multiples sont agencées en mosaïque pour former un champ d'image plus grand couvrant la zone d'intérêt.

11. Procédé selon la revendication 5, dans lequel l'image actuelle est alignée avec une ou plusieurs images précédemment capturées et traitées pour permettre une comparaison directe de changements de caractéristiques au cours du temps.

12. Procédé selon la revendication 5, dans lequel l'image unique est plénoptique ou mise au point à des profondeurs multiples et un fichier vidéo est créé qui permet à l'utilisateur de se déplacer par incrément sur un empilage de focale ou sélectionner une région à visualiser qui est mise au point.

13. Support de stockage informatique non transitoire comportant des instructions stockées sur celui-ci qui, lorsqu'elles sont exécutées, exécutent un procédé comprenant les étapes selon l'une quelconque des revendications 5 à 12.
